(19) Europäisches Patentamt

European Patent Office

Office européen des brevets

(11) EP 1 700 566 A1

(12) EUROPEAN PATENT APPLICATION

(43) Date of publication:
13.09.2006 Bulletin 2006/37

(51) Int Cl.:
$A61B\ 5/107^{(2006.01)}$     $A61F\ 2/06^{(2006.01)}$

(21) Application number: 06251223.1

(22) Date of filing: 08.03.2006

(84) Designated Contracting States:
AT BE BG CH CY CZ DE DK EE ES FI FR GB GR
HU IE IS IT LI LT LU LV MC NL PL PT RO SE SI
SK TR
Designated Extension States:
AL BA HR MK YU

(30) Priority: 10.03.2005 US 77267

(71) Applicant: GENERAL ELECTRIC COMPANY
Schenectady, NY 12345 (US)

(72) Inventors:
• Jenkins, John H.
Grand Prarie
TX 75050 (US)

• Morrien, Judith P.
3823 GJ Amersfoort (NL)
• Bouissaghouane, Khalid
3815 GA Amersfoort (NL)
• Solomon, Harry P.
Highland Park
IL 60035 (US)

(74) Representative: Pedder, James Cuthbert et al
London Patent Operation
General Electric International, Inc.
15 John Adam Street
London WC2N 6LU (GB)

(54) **System, method and computer instructions for estimating stent size**

(57)     Certain embodiments of the present invention provide a system, method and computer instructions for estimating stent size. In an embodiment, a stent size estimating system includes: an input module for inputting artery data; a calculation module for calculating stent size based on the artery data; and an output module for outputting stent data. The stent size estimating system may also include: an availability module for checking the availability of a stent; a selection module for selecting a stent based on output stent data; and/or a feedback module for inputting feedback, for example.

## FIG. 2

200

Input Artery data — 202

Calculate Stent Size — 204

Check Availability — 205

Output Stent Data — 206

Select Stent — 208

Input Feedback — 210

**Description**

The present invention generally relates to a system, method and computer instructions for estimating stent size. More particularly, the present invention relates to a system, method and computer instructions for inputting artery data, calculating stent size based on the artery data, and outputting stent data. The present invention also relates to a system, method and computer instructions for checking the availability of a stent, selecting a stent based on output stent data and/or inputting feedback.

Over time, an artery may become partially blocked by fatty material known as plaque. When an artery becomes partially blocked, blood flow through the artery is impaired. In order to restore regular blood flow, a stent may be introduced into the artery, displacing the accumulated plaque and restoring the artery to a properly functioning size. However, an artery may only function properly if a properly sized stent is introduced.

Determining proper stent size is of great importance. If a stent that is too large is introduced into an artery, the artery may rupture. On the other hand, if a stent that is too small is introduced into the artery, future surgery, introducing a new, larger stent, may be required, or other complications may arise. Consequently, in order to properly repair a partially blocked artery, there is a need for a cardiologist to first accurately determine the proper stent size.

Currently, Quantitative Coronary Analysis (QCA) systems are used to measure the width (chord length) of arteries. QCA systems can measure the chord length of an artery at any point along the analyzed section of the artery. For example, the chord length of an artery can be measured at the point where the artery is most severely blocked and/or at any other point.

However, no single measurement taken by a QCA system is alone sufficient to determine proper stent size. The measurements reported by the QCA system require manipulation in order to determine proper stent size. For example, many cardiologists believe that taking the chord length of an artery just prior to the point where blockage begins (proximal size or proximal chord length) and the chord length of the artery just past the point where blockage ends (distal size or distal chord length), and then dividing the result by two and multiplying by 90%, may provide the appropriate size for a properly sized stent.

Unfortunately, current QCA systems are not equipped to incorporate the above calculation. Current QCA systems only report artery data in the form of chord lengths, and then cardiologists are left to determine proper stent size. Many cardiologists execute the above calculation manually. However, manual calculation takes time and may result in errors. Further, a cardiologist may want to calculate stent size based on a range of chord lengths, thus resulting in numerous calculations. However, manually executing the calculation numerous times increases the amount of time spent on the task and increases the risk of error entering calculations. Further, spending time on manual calculations increases a cardiologists workload and delays action to correct a medical condition in a patient.

Thus, there is a need for a system, method and computer instructions for estimating stent size.

Certain embodiments of the present invention provide a system, method and computer instructions for estimating stent size. In an embodiment, a stent size estimating system includes: an input module for inputting artery data; a calculation module for calculating stent size based on the artery data; and an output module for outputting stent data. The stent size estimating system may also include: an availability module for checking the availability of a stent; a selection module for selecting a stent based on output stent data; and/or a feedback module for inputting feedback, for example.

In an embodiment, a method for estimating stent size includes: inputting artery data; calculating stent size based on the artery data; and outputting stent data. The method for estimating stent size may also include: checking the availability of a stent; selecting a stent based on output stent data; and/or inputting feedback, for example.

In an embodiment, a computer-readable storage medium includes a set of instructions for a computer directed to estimating stent size. The set of instructions includes: an input routine that allows artery data to be input; a calculation routine that calculates a stent size based on the artery data; and an output routine that outputs stent data. The set of instructions may also include: an availability routine that checks the availability of a stent; a selection routine that allows a stent to be selected based on output stent data; and/or a feedback routine that allows feedback to be input, for example.

The invention will now be described in greater detail, by way of example, with reference to the drawings, in which:-

Figure 1 illustrates a stent size estimating system used in accordance with an embodiment of the present invention.

Figure 2 illustrates a method for estimating stent size used in accordance with an embodiment of the present invention.

Figure 3 illustrates a set of computer instructions for estimating stent size used in accordance with an embodiment of the present invention.

Figure 4 illustrates an example of an input/output screen for inputting artery data and outputting stent data.

The foregoing summary, as well as the following detailed description of embodiments of the present invention, will be better understood when read in conjunction with the appended drawings. For the purpose of illustrating the invention, certain embodiments are shown in the drawings. It should be understood, however, that the present invention is not limited to the arrangements and instrumentality shown in the attached drawings.

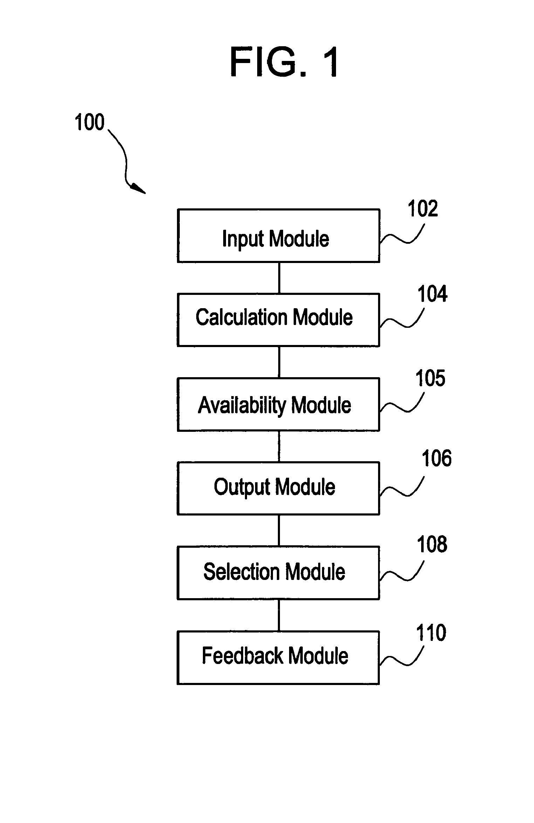

Figure 1 illustrates a stent size estimating system 100 that estimates stent sizes used in accordance with an embodiment of the present invention. The stent size estimating system 100 includes an input module 102, a calculation module 104, an availability module 105, an output module 106, a selection module 108, and a feedback module 110. The components of the system 100 may be implemented in many ways. For example, the components may be implemented in hardware and/or software. The components may be implemented separately and/or integrated in various combinations. Other desirable ways to implement the components of the system 100 may exist, as known to one of ordinary skill in the art.

The system 100 may also be implemented in many ways. For example, the system 100 may be integrated with existing applications that run on a Microsoft® platform and/or other platforms as an add-on product. In one implementation, the system 100 may be integrated with cardiac review stations such as the CA1000® station and/or the Innova® imaging system, as well as other cardiac review stations. Other desirable ways to implement the system 100 may exist, as known to one of ordinary skill in the art.

In the stent size estimating system 100, the input module 102 allows artery data to be input. As described below, the input module 102 may be configured to allow various types of artery data to be input from various sources. The artery data is then used by the calculation module 104 to estimate a proper stent size. As described below, the calculation module 104 may be configured to calculate a proper stent size based on various types of artery data and equations. Once a proper stent size is estimated, the availability of proper sized stents may be checked by the availability module 105. Once stent data, such as proper stent size and/or stent availability information, is prepared by the calculation module 104 and/or the availability module 105, stent data is output by the output module 106. As described below, the output module 104 may be configured to allow various types of stent data to be output in various ways. After stent data is output by the output module 106, a stent may be selected using the selection module 108. As described below, the selection module 108 may be configured to allow selection of a stent under various circumstances. Also, feedback regarding the accuracy of output stent data and/or any other aspect of the system may be input into the feedback module 110. As described below, the feedback module 110 may be configured to be accessed in various ways and/or so that various types of information may be input.

As mentioned above, the input module 102 may be configured to allow various types of artery data to be input. For example, the input module 102 may be configured to allow artery width data, also known as chord length, to be input. In one embodiment, the chord lengths of an artery at two specific points are of interest when estimating stent size. The two chord lengths are called the "proximal" chord length and the "distal" chord length. The "proximal" chord length is the chord length at a point in an artery preceding a blockage. The "distal" chord length is the chord length at a point in an artery following a blockage. The input module 102 may be configured to allow input of the proximal chord length and the distal chord length, as well as any other chord lengths of an artery.

The input module 102 may also be configured to allow artery type to be input, for example. The American College of Cardiology (ACC) defines various types of arteries. The input module 102 may be configured to allow these and other types of arteries to be input into the input module 102.

The input module 102 may be configured to allow artery data to be input in various ways. For example, the input module 102 may be configured to allow a doctor to manually enter artery data. Manual input allows a doctor or other healthcare practitioner to enter chord length data arrived at by any method, such as by using "calipering" techniques and/or any other technique.

The input module 102 may also be configured to allow artery data to be input from an artery measuring system, such as a Quantitative Coronary Analysis (QCA) system. QCA systems provide the proximal and distal chord lengths discussed above and may include artery type information as well. QCA systems may be interfaced using COM Objects and/or by other methods.

The input module 102 may also be configured to allow artery data to be input from structured reports that comply with the Digital Imaging Communications in Medicine (DICOM) standards. DICOM data input allows data recorded during a previous analysis of an artery to be input into the input module 102. Further, the input module 102 may also be configured to allow artery data to be input from other data sources, such as a library or a database.

The input module 102 may also be configured to allow artery data to be input from visual displays. For example, a listing of all artery types defined by the ACC may be displayed in a visual display, so that the artery type may be selected manually.

Other types of artery data may exist and other desirable ways to input artery data into the input module 102 may exist, as known to one of ordinary skill in the art.

As mentioned above, the artery data input into the input module 102 is used by the calculation module 104 to estimate a proper stent size. The calculation module 104 may be configured to calculate proper stent size in many ways.

One implementation of the calculation module 104 applies an equation to estimate a proper stent size (PSS). The equation requires that a proximal chord length (PCL) and a distal chord length (DCL), as defined above, be input into the input module 102. The equation is as follows: PPS = 0.9*(PCL + DCL)/2. The calculation module 106 may be configured to utilize other types of artery data and other equations to estimate proper stent size.

As mentioned above, once proper stent size is estimated, stent availability may be checked by the availability module 105. The availability module 105 may be configured to check various sources of stents, such as a database of stents available at a healthcare facility, such as a hospital or a clinic, where a patient is being treated. The availability module 105 may also be configured to check the availability of stents at all healthcare facilities within a certain distance of the healthcare facility where a patient is being treated. In one embodiment, the availability module 105 may be configured to check other sources for stent availability.

Once stent data, such as proper stent size and/or stent availability information, is prepared by the calculation module 104 and/or the availability module 105, stent data is output by the output module 106. The output module 106 may be configured to output various types of stent data, such as stent size and stent availability, for example. In an embodiment, the output module 106 may be configured to output other types of stent data.

The output module 106 may be configured to output information in many ways. For example, the output may be a visual display, an audio display, printed matter, a facsimile transmission, and/or electronic mail. Other desirable ways to configure the output module 108 to output stent data may exist, as known to one of ordinary skill in the art.

As mentioned above, after stent data is output by the output module 106, a stent may be selected using the selection module 108. The selection module 108 may be configured to allow selection of a stent from the inventory of available stents at the healthcare facility where a patient is being treated. The selection module 108 may also be configured to allow selection of a stent from the inventory of available stents at healthcare facilities within a certain distance of the healthcare facility where a patient is being treated. The selection module 108 may also be configured to allow selection of a stent to be ordered and delivered to the healthcare facility where a patient is being treated. Other desirable ways to configure the selection module 108 for stent selection may exist, as known to one of ordinary skill in the art.

As mentioned above, feedback regarding the accuracy of output stent data and/or any other aspect of the system 100 may be input into the feedback module 110. The feedback module 110 may be configured to accept various types of input. For example, the feedback module 110 may be configured to accept input regarding the accuracy of stent data output by the output module 106. The feedback module 110 may also be configured to accept input regarding the usability of the system 100. The feedback module 110 may also be configured to accept input regarding any other aspect of the system 100. In an embodiment, the feedback module 110 may be configured to accept other types of input, as known to one of ordinary skill in the art.

The feedback module 110 may be configured to be accessible in various ways. For example, the feedback module 110 may be configured to be accessible from any visual interface of the system 100. The feedback module 110 may also be configured to be accessible from the output that is output by the output module 106. It may also be desirable to configure the feedback module 110 to be accessible in other ways, as known to one of ordinary skill in the art.

In operation, the stent size estimating system 100 is used in connection with cardiac review stations, such as the CA1000® station and the Innova® imaging system. First, artery data is input into the input module 102. For example, chord lengths of an artery are input from a QCA system, or a cardiologist manually inputs chord lengths of an artery. The chord lengths are then used by the calculation module 104 to estimate proper stent size. For example, the calculation module 104 enters artery data into an equation for estimating stent size, such as the equation mentioned above. Once a proper stent size is estimated, the availability of proper sized stents may be checked by the availability module 105. For example, the availability module 105 checks the availability of stents at the healthcare facility where the patient is being treated by interfacing a database of available stents. Then, stent data is output by the output module 106. For example, the output module 106 outputs the estimated stent size and/or the availability of stents at the healthcare facility as a visual display. After stent data is output by the output module 106, a stent may be selected using the selection module 108. For example, the selection module is used to select an available stent from output in the form of a visual display. Also, feedback regarding the accuracy of output stent data and/or any other aspect of the system may be input into the feedback module 110. For example, input regarding the accuracy of stent size and/or stent availability information output by the output module 106 is input into the feedback module 110.

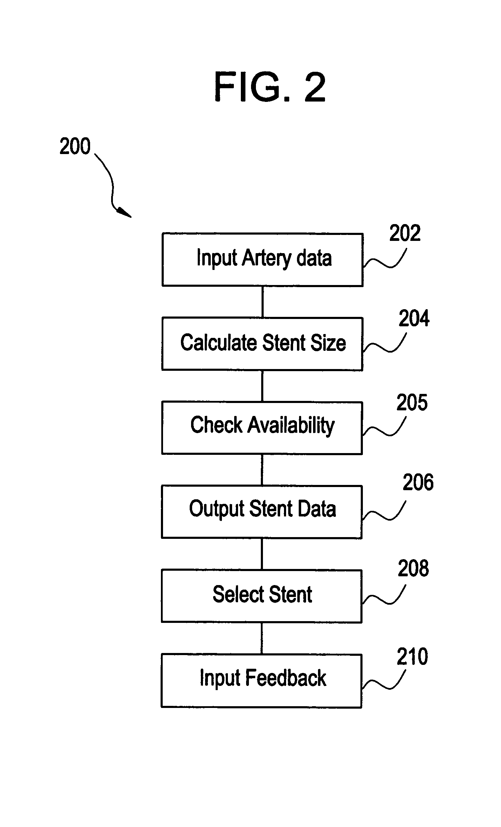

Figure 2 illustrates a method 200 for estimating stent size used in accordance with an embodiment of the present invention. At 202, artery data is input. For example, chord lengths of an artery are input from a QCA system or a cardiologist manually inputs chord lengths of an artery. At 204, stent size is estimated. For example, artery data is entered into an equation for estimating stent size, such as the equation mentioned above in relation to Figure 1. At 205, availability of proper sized stents is checked. For example, availability of stents at the healthcare facility where the patient is being treated may be checked by interfacing a database of available stents. At 206, stent data is output. For example, the estimated stent size and/or the availability of stents at the healthcare facility where the patient is being treated may be output to a visual display. At 208, a stent is selected. For example, an available stent may be selected from a visual display. At 210, feedback is input. For example, input regarding the accuracy of stent size and/or stent availability information output at 206 may be input.

When a cardiologist is faced with repairing a partially blocked artery, the cardiologist must first estimate a proper stent size and then insert the stent into the artery. However, it may be difficult to estimate a proper stent size in a timely manner because current artery measurement systems do not accurately estimate proper stent sizes. Applying the method 200, as described above and/or in light of the description of Figure 1, may aid cardiologists in quickly estimating a proper stent size by allowing the cardiologist to utilize an accepted equation that estimates stent size based on artery measurements that are input manually and/or are input from electronic sources, such as QCA systems.

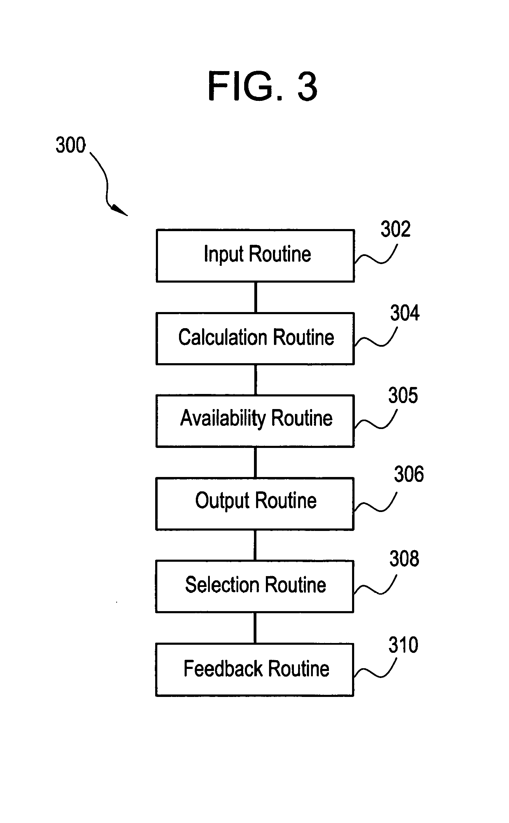

Figure 3 illustrates a set of computer instructions 300 for estimating stent size used in accordance with an embodiment of the present invention. The set of computer instructions 300 for estimating stent size includes an input routine 302, a calculation routine 304, an availability routine 305, an output routine 306, a selection routine 308, and a feedback routine 310. The set of computer instructions 300 may be implemented on cardiac review stations such as the CA1000® station and/or the Innova® imaging system, as well as other cardiac review stations, for example.

In the set of computer instructions 300 for estimating stent size, the input routine 302 allows artery data to be input. The calculation routine 304 calculates a proper stent size. The availability routine 305 checks the availability of stents. The output routine 306 outputs stent data. The selection routine 308 allows a stent to be selected. The feedback module 110 allows feedback to be input.

In an embodiment, the input routine 302, the calculation routine 304, the availability routine 305, the output routine 306, the selection routine 308, and the feedback routine 310, may perform functions similar to the input module 302, the calculation module 304, the availability module 305, the output module 306, the selection module 308, and the feedback module 310, respectively, as described above in relation to Figure 1.

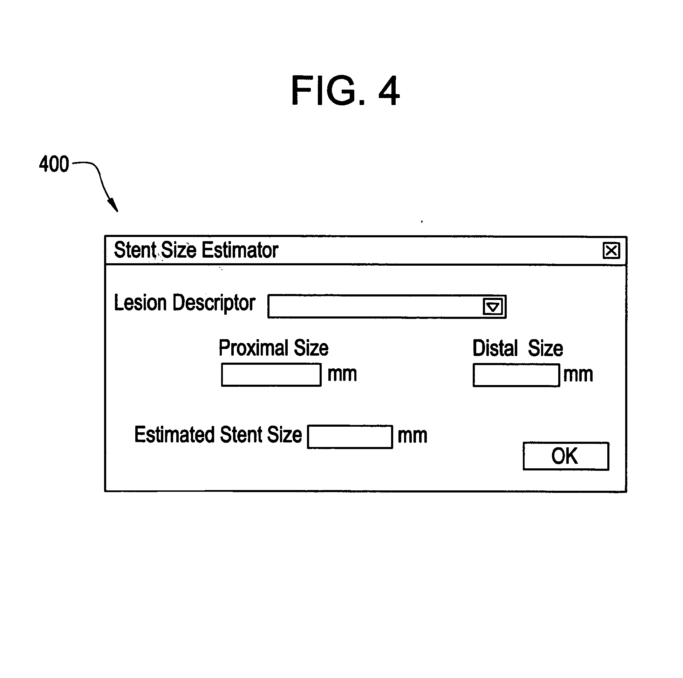

Figure 4 illustrates an input/output screen used in accordance with an embodiment of the present invention for inputting artery data and outputting stent data. The input/output screen allows three types of artery data, for example, to be input: lesion descriptor data, proximal size data, and distal size data. The lesion descriptor data field allows artery type to be selected from a pull-down list. The pull-down list may be populated with artery types defined by the American College of Cardiology (ACC), for example. The pull-down list may also be populated with artery types that are manually input.

The proximal size and distal size fields allow artery size data to be input. Proximal size and distal size are equivalent to proximal chord length and distal chord length, as described previously in relation to Figure 1 above, for example. Both fields may be populated automatically from an electronic source, such as a QCA system, and/or manually, for example.

The input/output screen also has a field for outputting estimated stent size. The estimated stent size field outputs an estimated stent size based on data input into the input fields of the input/output screen. The estimated stent size field may output an estimated stent size based on any combination of data input into the input fields of the input/output screen.

Thus, certain embodiments of the present application provide a system, method and computer instructions for estimating stent size in connection with cardiac review stations, such as the CA1000® station and/or the Innova® imaging system, for example. Certain embodiments estimate stent size based on proximal and distal chord data input manually and/or from an electronic source, such as a QCA system, for example. Certain embodiments take artery type, as defined by the ACC, into account when estimating stent size, for example. Certain embodiments check the availability of stents by interfacing with a healthcare facility database(s) that contains stent availability information, for example. Certain embodiments output stent data including stent size and stent availability data, for example. Certain embodiments allow a stent to be selected based on output stent data, for example. Certain embodiments allow feedback regarding output stent data to be input, for example.

**Claims**

1.  A system (100) for estimating stent size comprising:

    an input module (102) for inputting artery data;
    a calculation module (104) for calculating stent size based on said artery data; and
    an output module (106) for outputting stent data.

2.  The system (100) of claim 1, further comprising at least one of:

    an availability module (105) for checking the availability of a stent;
    a selection module (108) for selecting a stent based on said stent data; and
    a feedback module (110) for inputting feedback.

3. The system (100) of claim 2, wherein said stent data comprises at least one of:

> stent size; and
> stent availability information.

4. The system (100) of claim 1, wherein said artery data comprises at least one of:

> proximal chord data;
> distal chord data; and
> type of artery data.

5. The system (100) of claim 1, wherein said artery data comprises proximal chord length and distal chord length; and wherein said calculation module (104) calculates said stent size based on the following formula:

$$0.9*(\text{proximal chord length} + \text{distal chord length})/2.$$

6. A method (200) for estimating stent size comprising:

> inputting artery data;
> calculating stent size based on said artery data; and
> outputting stent data.

7. The method (200) of claim 6, further comprising at least one of:

> checking the availability of a stent;
> selecting a stent based on said stent data; and
> inputting feedback.

8. The method (200) of claim 7, wherein said stent data comprises at least one of:

> stent size; and
> stent availability information.

9. The method (200) of claim 6, wherein said artery data comprises at least one of:

> proximal chord data;
> distal chord data; and
> type of artery data.

10. The method (200) of claim 6, wherein said artery data comprises proximal chord length and distal chord length; and wherein said stent size is calculated based on the following formula: 0.9*(proximal chord length + distal chord length) /2.

# FIG. 1

100

| Input Module | 102 |
|---|---|

| Calculation Module | 104 |
|---|---|

| Availability Module | 105 |
|---|---|

| Output Module | 106 |
|---|---|

| Selection Module | 108 |
|---|---|

| Feedback Module | 110 |
|---|---|

# FIG. 2

200

| Input Artery data | 202 |
| Calculate Stent Size | 204 |
| Check Availability | 205 |
| Output Stent Data | 206 |
| Select Stent | 208 |
| Input Feedback | 210 |

# FIG. 3

300

| Input Routine | 302 |
| --- | --- |
| Calculation Routine | 304 |
| Availability Routine | 305 |
| Output Routine | 306 |
| Selection Routine | 308 |
| Feedback Routine | 310 |

# FIG. 4

400

| Stent Size Estimator | ⊠ |
|---|---|

**Lesion Descriptor** [　　　　　　　▽]

**Proximal Size**　　　　　　　　**Distal Size**
[　　　　]mm　　　　　　　　[　　　　]mm

**Estimated Stent Size** [　　　　] mm

[ OK ]

EP 1 700 566 A1

European Patent Office

EUROPEAN SEARCH REPORT

Application Number

EP 06 25 1223

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 782 284 B1 (SUBRAMANYAN KRISHNA ET AL) 24 August 2004 (2004-08-24) * column 1, line 49 - line 50; figures 4,9,10 * * column 4, line 26 - line 27 * * column 8, line 15 - line 64 * | 1-10 | INV. A61B5/107 A61F2/06 |
| A | "RX ACCULINK Carotid Stent System" GUIDANT, [Online] 18 August 2004 (2004-08-18), pages 26-27, XP002387696 Internet Retrieved from the Internet: URL:http://www.guidant.com/products/TemplatePDFs/ACCULINK_RX.pdf> [retrieved on 2006-06-28] * page 26 - page 27 * | 1-10 | |
| A | WO 03/049794 A (BIOSENSORS INTERNATIONAL PTE LTD; KOH, SIAM, SOON, PHILIP; PING, YE; S) 19 June 2003 (2003-06-19) * abstract * | 1-10 | TECHNICAL FIELDS SEARCHED (IPC) A61B A61F |
| A | US 2002/023843 A1 (CHERKES DAVID) 28 February 2002 (2002-02-28) * abstract * | 1-10 | |

The present search report has been drawn up for all claims

| Place of search | Date of completion of the search | Examiner |
|---|---|---|
| The Hague | 28 June 2006 | Schwenke, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

11

**EP 1 700 566 A1**

## ANNEX TO THE EUROPEAN SEARCH REPORT
## ON EUROPEAN PATENT APPLICATION NO.

EP 06 25 1223

This annex lists the patent family members relating to the patent documents cited in the above-mentioned European search report.
The members are as contained in the European Patent Office EDP file on
The European Patent Office is in no way liable for these particulars which are merely given for the purpose of information.

28-06-2006

| Patent document cited in search report | | Publication date | Patent family member(s) | | Publication date |
|---|---|---|---|---|---|
| US 6782284 | B1 | 24-08-2004 | EP | 1469778 A2 | 27-10-2004 |
| | | | JP | 2005510280 T | 21-04-2005 |
| | | | WO | 03045244 A2 | 05-06-2003 |
| WO 03049794 | A | 19-06-2003 | AU | 2002366628 A1 | 23-06-2003 |
| US 2002023843 | A1 | 28-02-2002 | AU | 6780901 A | 30-01-2002 |
| | | | EP | 1315850 A1 | 04-06-2003 |
| | | | WO | 0206567 A1 | 24-01-2002 |

For more details about this annex : see Official Journal of the European Patent Office, No. 12/82